# EUROPÄISCHE PATENTSCHRIFT

(11) **EP 2 667 804 B1**
(45) Veröffentlichungstag und Bekanntmachung des Hinweises auf die Patenterteilung: **16.09.2015**
(21) Anmeldenummer: 12705988.9
(22) Anmeldetag: 23.01.2012
(51) Int. Cl.: A61B 17/34, A61M 25/01

(54) **SUPRAPUBISCHE SICHERHEITSKANÜLE**
SUPRAPUBIC SAFETY CANNULA
CANULE DE SÉCURITÉ SUPRAPUBIENNE

(30) Priorität: 26.01.2011 DE 102011009482
(43) Veröffentlichungstag der Anmeldung: 04.12.2013
(73) Patentinhaber: Haindl, Hans, 30974 Wennigsen (DE)
(72) Erfinder: Haindl, Hans, 30974 Wennigsen (DE)
(74) Vertreter: Vossius & Partner Patentanwälte Rechtsanwälte mbB
(86) Internationale Anmeldenummer: PCT/EP2012/050966
(87) Internationale Veröffentlichungsnummer: WO 2012/101089

(56) Entgegenhaltungen:
- FR-A5- 2 129 116

## Beschreibung

Die vorliegende Anmeldung betrifft eine Sicherheitskanüle, insbesondere eine Sicherheitskanüle zur Punktion von Körperhohlräumen, z.B. eine suprapubische Sicherheitskanüle.

Es gibt in verschiedenen Bereichen der Medizin das Problem, dass ein Katheter durch eine Kanüle in einen Körperhohlraum eingeführt wird und dass danach die Kanüle über den fest konektierten Ansatz des Katheters nicht mehr entfernt werden kann. Im Stand der Technik sind verschiedene Lösungen bekannt, mit diesem Problem umzugehen. So wurden beispielsweise bei zentralvenösen Kathetern über der Kanüle Kunststoff-Schutztaschen angebracht, um eine Beschädigung des Katheters oder eine Verletzung des Patienten oder Anwenders zu verhindern.

Auch aus dem Bereich der Urologie ist das oben genannte Problem bekannt. Um eine längerfristige künstliche Harnableitung zu ermöglichen, hat sich die suprapubische Punktion der Blase (d. h. durch die Bauchdecke hindurch) durchgesetzt. Man verwendet hierzu sogenannte suprapubische Katheter, deren Infektionsrate niedriger ist als die von Kathetern, die durch die Harnröhre eingeführt werden. Auch bei diesen suprapubischen Kathetern tritt das Problem auf, dass die Kanüle, wenn der Katheter eingeschoben ist und die Kanüle aus dem Katheter wieder herausgezogen worden ist, sich über den Ansatz des Katheters nicht entfernen lässt. Die Kanüle kann aber nicht auf dem Katheter belassen werden, da sie leicht zu einer Beschädigung des Katheters und/oder zu einer Verletzung des Patienten führen würde.

Um ein Entfernen der Kanüle vom Katheter zu ermöglichen, ist es bekannt, die Kanüle nach dem Gebrauch zu spalten. Der Anwender zieht beispielsweise an zwei Griffen in entgegengesetzte Richtungen und spaltet damit die Kanüle in ihrer Längsrichtung auf. Die beiden Kanülenhälften können dann einfach von dem Katheter entfernt werden. Solche Kanülen sind beispielsweise aus DE 43 16 793 C1, DE 2 104 211 C1, DE 698 37 667 T2 und DE 10 2005 015 556 A1 bekannt. Beispiele für spaltbare Kanülen sind gerollte Kanülen, die an einer Seite einen offenen Spalt haben und an der anderen Seite vorgeschwächt sind, mechanisch an zwei Seiten vorgeschwächte Kanülen sowie Laser-perforierte Kanülen.

Der Vorgang des Spaltens erfordert allerdings erhebliche Kraft und beinhaltet für den Anwender ein hohes Verletzungsrisiko. Die Spaltkanten der Kanülen sind häufig mit scharfen Graten bedeckt, die bei Berührung zu Schnittverletzungen führen können.

Eine aus dem Stand der Technik bekannte Alternative, bei der auf das Spalten der Kanüle verzichtet werden kann, ist die sogenannte Rinnenkanüle, aus der sich der Katheter seitlich entnehmen lässt. Beispiele für solche Rinnenkanülen sind in der EP 0 499 147 B1 und der DE 41 03 977 A1 beschrieben. Die US 3,545,443 sowie die DE 33 47 150 A1 beschreiben Kanülen, die aus zwei längsgeschnittenen Hülsen bestehen, die einander überlappen.

Die US 7,708,721 B2 beschreibt eine Kanüle mit gegeneinander verschiebbaren Innen- und Außenenhülsen gemäß der Präambel des Anspruchs 1.

Allerdings lässt sich die Kanülenspitze der Innenhülse in der zurückgezogenen Sicherheitsposition nicht fixieren, so dass nicht verhindert werden kann, dass die Kanülenspitze wieder austritt und für Verletzungen sorgt. Ferner lässt sich die in der US 7,708,721 B2 beschriebene Gewindeführung nicht oder nur mit nicht vertretbarem Aufwand technisch umsetzen.

Auch wenn die zuletzt genannten Druckschriften das Verletzungsrisiko dahingehend verringern, dass keine Spaltkanten mit scharfen Graten entstehen, eliminieren die darin vorgeschlagenen Lösungsansätze nicht die Gefahr, sich an der extrem scharfen Punktionsspitze der Kanüle zu verletzen und ggf. zu infizieren. Nach der geltenden Unfallverhütungsvorschrift TRBA 250 sind Krankenhausbetreiber gehalten, ihren Angestellten verletzungssichere Kanülensysteme zur Verfügung zu stellen. Beispielsweise im Bereich der Urologie für die suprapubische Blasenfunktion steht bislang kein völlig verletzungssicheres System zur Verfügung.

Es ist daher eine Aufgabe der vorliegenden Erfindung, eine Sicherheitskanüle, insbesondere eine Sicherheitskanüle zur Punktion von Körperhohlräumen, bereitzustellen, die das Verletzungs- und Infektionsrisiko bekannter Systeme weiter minimiert. Diese Aufgabe wird mit einer Kanüle gemäß Anspruch 1 gelöst.

Erfindungsgemäß wird eine Kanüle zur Punktion von Körperhohlräumen mit einer äußeren und einer inneren längsgeschnittenen Hülse mit jeweils proximalen und distalen Enden bereitgestellt. Die innere Hülse ist innerhalb der äußeren Hülse angeordnet und lässt sich innerhalb der äußeren Hülse in Längsrichtung verschieben. Ferner lassen sich die innere und die äußere Hülse derart gegen einander verdrehen, dass der Längsschnitt der inneren Hülse in einer ersten Drehposition durch die äußere Hülse vollständig abgedeckt wird und die Längsschnitte von innerer und äußerer Hülse in einer zweiten Drehposition übereinander zu liegen kommen. In einer ersten Drehposition bilden die beiden Hülsen eine vollständig geschlossene Kanüle, die zur Punktion von Körperhohlräumen geeignet ist. In der zweiten Position sind die beiden Längsschnitte der Hülsen deckungsgleich angeordnet, so dass ein Katheter oder Katheterschlauch, der sich innerhalb der inneren Hülse befindet, durch diese Längsschnitte aus der Hülse entfernt werden kann. Dabei beruht die Erfindung auf der Idee, dass nur die innere Hülse eine scharf geschliffene Punktionsspitze aufweist, wohingegen das distale Ende der äußeren Hülse derart ausgestaltet ist, dass es nicht zu Verletzungen führen kann, und dass diese scharf geschliffene Punktionsspitze der inneren Hülse in dem Moment, in dem die Kanüle von dem Katheter oder Katheterschlauch abgenommen wird, vollständig durch die äußere Hülse geschützt ist. Entsprechend weist die innere Hülse erfindungsgemäß an ihrem distalen Ende eine Punktionsspitze auf, die vollständig in die äußere Hülse zurückgezogen ist, wenn sich die innere Hülse in der zweiten Drehposition befindet.

Dadurch wird sichergestellt, dass die scharfe Punktionsspitze bereits beim Abnehmen der Kanüle von dem Katheter oder Katheterschlauch für den Anwender bzw. das Klinikpersonal nicht mehr zugänglich ist. Eine Verletzung und möglicherweise Infektion durch die Punktionsspitze wird damit auf äußerst wirkungsvolle Weise vermieden. Erfindungsgemäß werden zwei bevorzugte Varianten für diesen Schutzmechanismus beschrieben.

Gemäß einer ersten bevorzugten Ausführungsform kann die innere Hülse nur dann in die zweite Position gedreht werden, wenn die Punktionsspitze der inneren Hülse bereits vollständig in die äußere Hülse zurückgezogen ist. Entsprechend kann die Kanüle durch eine zweistufige Bewegung geöffnet werden: Zunächst muss die innere Hülse innerhalb der äußeren Hülse soweit zurückgezogen werden, dass die Punktionsspitze der inneren Hülse vollständig von der äußeren Hülse abgedeckt bzw. geschützt wird. Erst in dieser Position erlaubt die erfindungsgemäße Kanüle ein Verdrehen der beiden Hülsen gegeneinander in die zweite Position, bei der die Längsschnitte der beiden Hülsen übereinander zu liegen kommen. Bevorzugt werden die beiden Bewegungen mit Hilfe von Griffen oder dergleichen bewerkstelligt, die zunächst in Längsrichtung gegeneinander bewegt werden müssen und dann gegeneinander verdreht werden können.

Entsprechend weist die Kanüle bevorzugt an ihrem proximalen Ende zwei Griffabschnitte oder Flügel auf, die eine Drehung der inneren Hülse blockieren, so lange sich die Punktionsspitze der inneren Hülse distal aus der äußeren Hülse heraus erstreckt. Bevorzugt ist dabei ein erster Griffabschnitt oder Flügel mit der inneren Hülse verbunden und ein zweiter Griffabschnitt oder Flügel mit der äußeren Hülse verbunden.

Gemäß einer zweiten bevorzugten Ausführungsform bewirkt eine Drehung der inneren Hülse in die zweite Position, dass die Punktionsspitze der inneren Hülse vollständig in die äußere Hülse zurückgezogen wird. Mit anderen Worten sind bei dieser Ausführungsform die beiden Bewegungen, d. h. die Verschiebung in Längsrichtung einerseits und die Drehung der Hülsen gegeneinander andererseits, miteinander gekoppelt, so dass ein Verdrehen der Hülsen gegeneinander zugleich ein Verschiebung der Hülsen in Längsrichtung bewirkt und vice versa. Bevorzugt sind die Dreh- und die Verschiebebewegung der Hülsen mittels eines Gewindes hinreichend großer Steigung miteinander gekoppelt, es sind aber auch andere Koppelungsmöglichkeiten im Rahmen der Erfindung anwendbar. Das Gewinde ist bevorzugt an den Griffabschnitten oder anderen proximalen Bereichen vorgesehen, die einen gegenüber dem Kanülendurchmesser vergrößerten Durchmesser aufweisen. Dadurch lässt sich das Gewinde einfacher und kostengünstiger herstellen als wenn das Gewinde in die Innen- bzw. Außenseite der Kanülen geschnitten werden müsste.

Bevorzugt erfolgt das Verdrehen der Hülsen gegen einander mittels an diesen angebrachten Griffabschnitten oder Flügeln. Entsprechend weist die Kanüle bevorzugt an ihrem proximalen Ende einen ersten, mit der inneren Hülse verbundenen Griffabschnitt und einen zweiten, mit der äußeren Hülse verbundenen Griffabschnitt auf, wobei eine relative Drehbewegung der Griffabschnitte bewirkt, dass die Punktionsspitze der inneren Hülse vollständig in die äußere Hülse zurückgezogen wird und die Längsschnitte von innerer und äußerer Hülse übereinander zu liegen kommen.

Im Falle beider Ausführungsformen ist es bevorzugt, dass das distale Ende der äußeren Hülse im Wesentlichen stumpf ist. Insbesondere soll das distale Ende der äußeren Hülse derart ausgebildet sein, dass Verletzungen an der äußeren Hülse vermieden werden.

Es ist ferner vorgesehen, dass die innere und äußere Hülse und/oder die beiden Griffabschnitte in der zweiten Position miteinander verrasten. Dadurch soll verhindert werden, dass sich die beiden Hülsen beim Abnehmen der Kanüle vom Katheter (oder später) zurück drehen und gegeneinander verschieben, was die Punktionsspitze der inneren Hülse ungeschützt hervortreten ließe. Bevorzugt erfolgt das Verrasten unlösbar, so dass die Kanüle nur als Einwegkanüle verwendet werden kann.

Es ist ferner bevorzugt, dass ein vollständiges Zurückdrehen der inneren Hülse aus der zweiten Position in die erste Position verhindert wird. Dies kann beispielsweise dadurch erfolgen, dass sich die innere Hülse (die leicht vorgespannt sein kann) in der zweiten Position aufweitet, was ein Zurückdrehen in die erste Position unmöglich macht, da die innere Hülse mit ihrem aufgeweiteten Durchmesser nicht mehr von der äußeren Hülse aufgenommen werden kann.

Es ist ferner bevorzugt, dass die Innenfläche der äußeren Hülse an ihrem distalen Ende zwei oder mehr Stabilisierungselemente aufweist, die die innere Hülse während der Punktion gegenüber den dabei auftretenden Druck- und Torsionskräften stabilisieren. Bei den Stabilisierungselementen kann es sich um Einkerbungen, Sicken oder Vorsprünge an der Innenfläche der äußeren Hülse handeln.

In einer weiteren bevorzugten Ausführungsform ist zwischen der ersten und zweiten Drehposition eine dritte Drehposition vorgesehen, in der die Punktionsspitze bereits vollständig in die äußere Hülse zurückziehbar oder zurückgezogen ist, die Längsschnitte von innerer und äußerer Hülse aber noch nicht übereinander zu liegen kommen. Dabei wird bevorzugt ein vollständiges Zurückdrehen der inneren Hülse aus der dritten Position in die erste Position verhindert.

Sämtliche mit Bezug auf die zweite Position beschriebenen Merkmale können zusätzlich oder alternativ auch für die dritte Position vorgesehen sein. So kann beispielsweise eine Drehung der inneren Hülse in die dritte Position bewirken, dass die Punktionsspitze der inneren Hülse vollständig in die äußere Hülse zurückziehbar wird.

Bevorzugt sind die Dreh- und die Verschiebebewegung der Hülsen mittels eines Gewindes miteinander gekoppelt. Hierbei weist die Kanüle an ihrem proximalen Ende bevorzugt einen ersten, mit der inneren Hülse verbundenen Griffabschnitt und einen zweiten, mit der äußeren Hülse verbundenen Griffabschnitt auf, wobei eine relative Drehbewegung der Griffabschnitte bewirkt, dass die Punktionsspitze der inneren Hülse zunächst in der dritten Drehposition vollständig in die äußere Hülse zurückgezogen wird und dann die Längsschnitte von innerer und äußerer Hülse in der zweiten Drehposition übereinander zu liegen kommen. Das Gewinde ist bevorzugt an den Griffabschnitten oder anderen proximalen Bereichen vorgesehen, die einen gegenüber dem Kanülendurchmesser vergrößerten Durchmesser aufweisen. Dadurch lässt sich das Gewinde einfacher und kostengünstiger herstellen als wenn das Gewinde in die Innen- bzw. Außenseite der Kanülen geschnitten werden müsste.

Es ist ferner bevorzugt, dass die äußere Hülse und der damit verbundene Griffabschnitt, bevorzugt einteilig, aus Kunststoff hergestellt sind. Unter anderem lässt sich die Gewindestruktur in den Griffabschnitten besonders einfach aus Kunststoff herstellen.

Gemäß einer bevorzugten Ausführungsform weist der mit der inneren Hülse verbundene Griffabschnitt eine Druckplatte auf. Diese dient dazu, dass der Anwender z.B. mittels der Handfläche oder des Daumens beim Punktieren flächig Druck ausüben kann. Dies erleichtert dem Anwender das Punktieren und ermöglicht somit eine sicherere Arbeitsweise. Da gegebenenfalls die innere Hülse einen Katheter o.ä. aufnehmen können soll, weist die Druckplatte bevorzugt einen Schlitz bzw. eine Führung für einen Katheter auf, damit verhindert werden kann, dass der Katheter z.B. während des Punktierens zwischen Druckplatte und Handfläche oder Daumen eingeklemmt oder gequetscht werden kann.

Die vorliegende Anmeldung betrifft ferner ein Kit mit einer wie oben beschriebenen Kanüle und einem Katheter, der in der inneren Hülse der Kanüle aufgenommen werden kann, wobei der Katheter in der zweiten Kanülenposition durch die übereinander liegenden Längsschnitte der Hülsen aus der Kanüle entfernt werden kann. Hierzu sollte vorzugsweise die Dimensionierung der Längsschnitte der Hülsen an den Durchmesser des Katheters angepasst sein. Im Falle eines Katheters aus relativ hartem Material sollten die Längsschnitte bevorzugt mindestens so groß sein wie der Durchmesser des Katheters. Besteht der Katheter jedoch aus einem weicheren Material, lässt sich dieser auch durch Schlitze entfernen, die kleiner als der Katheterdurchmesser sind, indem die Elastizität des Katheters ausgenutzt wird.

Dem Fachmann ist klar, dass die hierin beschriebenen Sicherheitsmerkmale sämtlich auch für beliebige andere Kanülen, insbesondere Punktionskanülen, zum Einsatz kommen können.

Bevorzugte Ausführungsformen der vorliegenden Anmeldung werden nachfolgend mit Bezug auf die Figuren näher beschrieben. Es zeigen:
- Fig. 1-5: eine erfindungsgemäße Kanüle gemäß einer ersten Ausführungsform in perspektivischer Ansicht (a) und in Schnittansicht (b) bei verschiedenen Relativpositionen von inneren und äußerer Hülse;
- Fig. 6a-6f: Detailansichten der Ausführungsform gemäß Figuren 1-5 bei verschiedenen Relativpositionen von inneren und äußerer Hülse;
- Fig. 7-10: eine erfindungsgemäße Kanüle gemäß einer zweiten Ausführungsform in perspektivischer Ansicht (a) und in Schnittansicht (b);
- Fig. 11a-11f: das Funktionsprinzip der erfindungsgemäßen Kanüle;
- Fig. 12a-12d: die Wirkungsweise der Kanülenhülsen gemäß einer bevorzugten Ausführungsform;
- Fig. 13: ein erfindungsgemäßes Detail einer erfindungsgemäßen Kanülenspitze in Schnittansicht (a) und in perspektivischer Ansicht (b);
- Fig. 14: eine erfindungsgemäße Kanüle gemäß einer dritten Ausführungsform;
- Fig. 15a-15f: die Kanüle gemäß Fig. 14 in erster (Fig. 15a und 15b), dritter (Fig. 15c und 15d) und zweiter (Fig. 15e und 15f) Drehposition;
- Fig. 16a-16f: die Kanüle gemäß Fig. 14 in erster (Fig. 16a und 16b), dritter (Fig. 16c und 16d) und zweiter (Fig. 16e und 16f) Drehposition;
- Fig. 17: die Kanüle gemäß Fig. 14 vor deren Montage;
- Fig. 18-18a: die Kanüle gemäß Fig. 14 nach der Montage, aber vor ihrem Einsatz;
- Fig. 19a-19e: die Kanüle gemäß Fig. 14 in erster (Fig. 19a), dritter (Fig. 19b und 19c) und zweiter (Fig. 19d und 19e) Drehposition;
- Fig. 20a-20c: den ersten Griffabschnitt der Kanüle gemäß Fig. 14;
- Fig. 20d-20f: die Kanüle gemäß Fig. 14 in der Prämontageposition (Fig. 20d) sowie in der ersten Drehposition (Fig. 20e und 20f);
- Fig. 21a-21c: eine erfindungsgemäße Kanüle gemäß einer vierten Ausführungsform; und
- Fig. 22a-22f: eine erfindungsgemäße Kanüle gemäß einer fünften Ausführungsform.

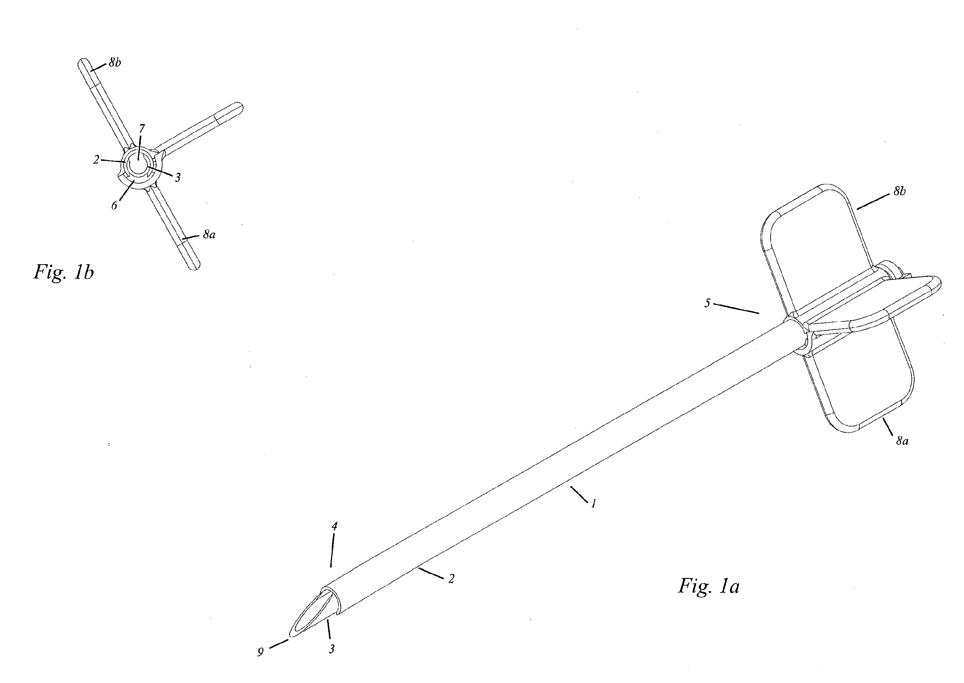

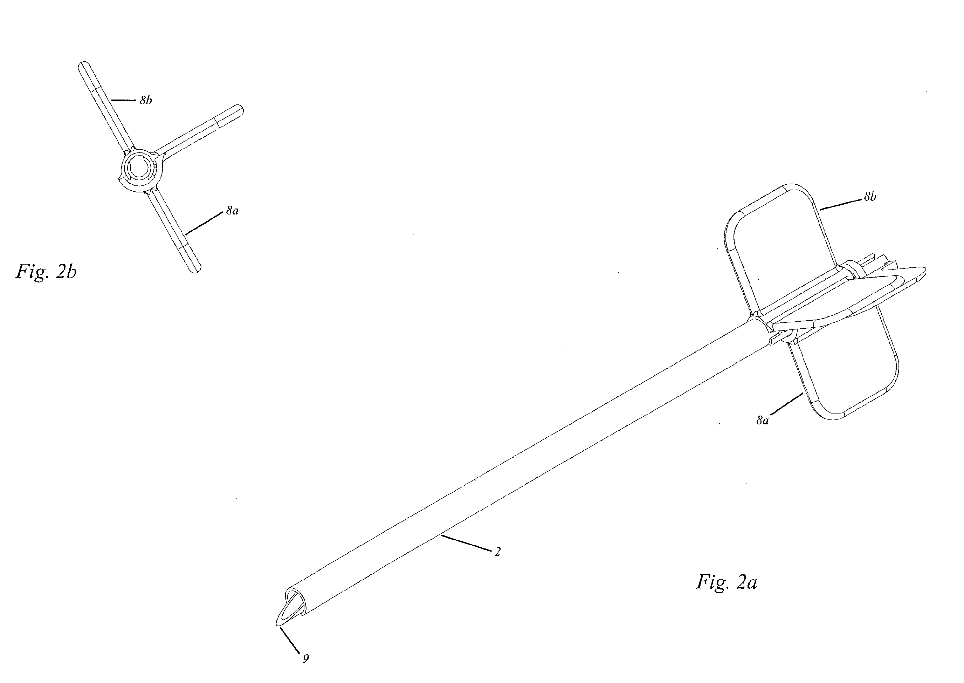

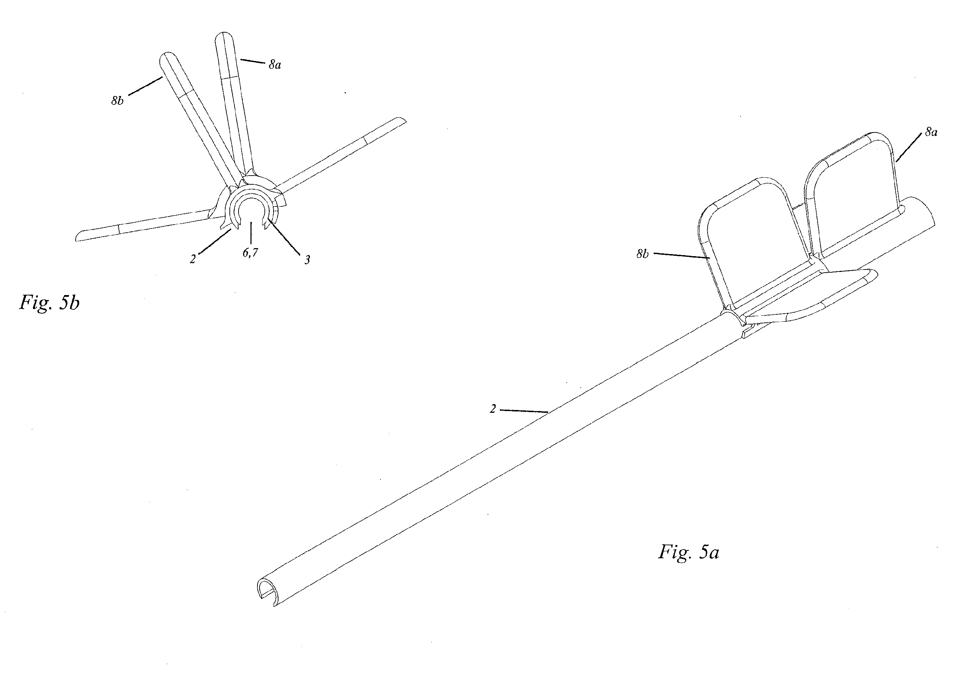

Figuren 1-5 zeigen eine erfindungsgemäße Kanüle gemäß einer ersten Ausführungsform. Fig. 1 zeigt die Kanüle in der ersten Drehposition und Fig. 5 die Kanüle in der zweiten Drehposition. Die Figuren 2-4 illustrieren den Bewegungsablauf zwischen der ersten und der zweiten Drehposition. Die Kanüle 1 zur Punktion von Körperhohlräumen weist eine äußere Hülse 2 mit einem Längsschnitt 6 und eine innere Hülse 3 mit einem Längsschnitt 7 (vgl. die Schnittansicht in Fig. 1b) auf. Die Hülsen besitzen ein proximales Ende 5 und ein distales Ende 4. Die innere Hülse 3 ist innerhalb der äußeren Hülse 2 in Längsrichtung verschiebbar angeordnet und kann in der äußeren Hülse 2 derart gedreht werden, dass der Längsschnitt 7 der inneren Hülse 3 in der in Fig.1a und 1b dargestellten ersten Drehposition durch die äußere Hülse 3 vollständig abgedeckt wird. In Fig. 1b ist der Versatz zwischen den beiden Längsschnitten 6 und 7 der äußeren und inneren Hülse deutlich zu erkennen. In der dargestellten Position ragt die Punktionsspitze 9 am distalen Ende 4 der inneren Hülse 3 distal aus der äußeren Hülse 2 hervor. Mittels dieser scharf geschliffenen Punktionsspitze 9 lässt sich ein Körperhohlraum, beispielsweise die Blase, punktieren. Die Kanüle 1 gemäß der ersten Ausführungsform weist an ihrem proximalen Ende 5 zwei Griffabschnitte oder Flügel 8a und 8b auf, die eine Drehung der inneren Hülse 3 blockieren, so lange sich die Punktionsspitze 9 der inneren Hülse 3 distal aus der äußeren Hülse heraus erstreckt.

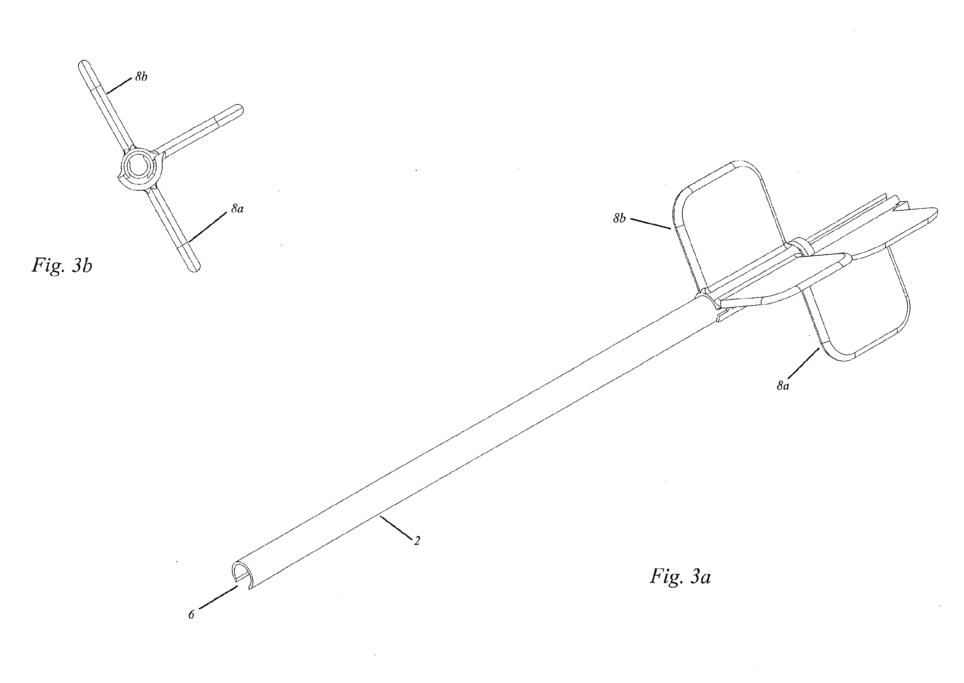

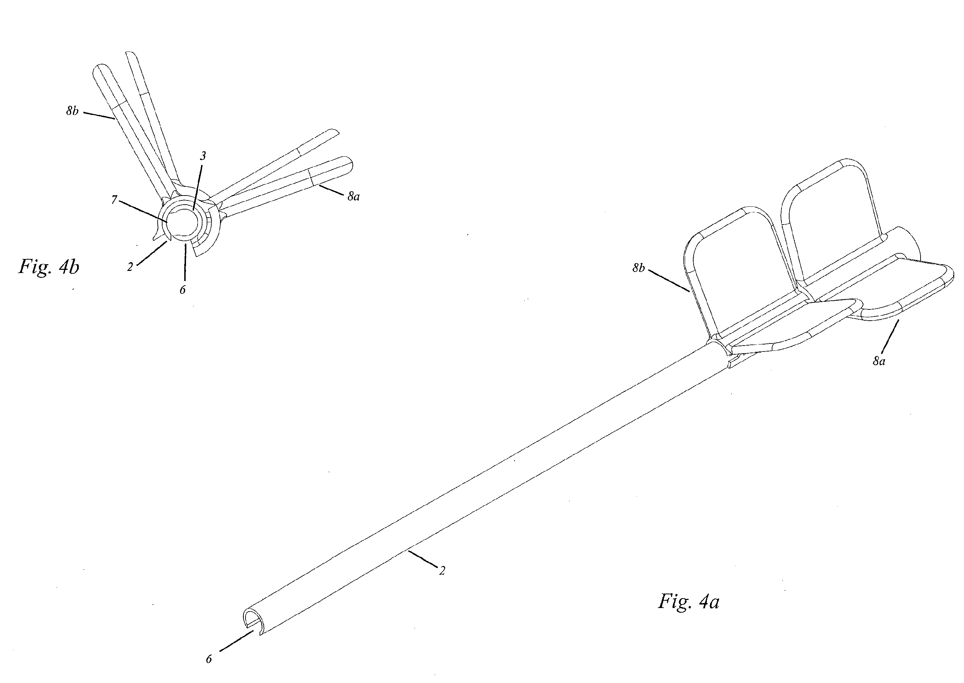

Um die beiden Hülsen gegeneinander verdrehen zu können, so dass die Längsschnitte 6 und 7 der beiden Hülsen übereinander zu liegen kommen, wie in den Figuren 5a und 5b zu sehen, muss zunächst die innere Hülse 3 proximal gegenüber der äußeren Hülse 2 zurückgezogen werden. Dies kann dadurch erzielt werden, dass der erste Griffabschnitt 8a, der mit der inneren Hülse 3 verbunden ist, gegenüber dem zweiten Griffabschnitt 8b, der mit der äußeren Hülse 2 verbunden ist, proximal verschoben wird, wie in den Figuren 2 und 3 dargestellt. In der in Fig. 3a gezeigten Position blockieren sich die beiden Griffabschnitte oder Flügel 8a und 8b nicht mehr gegenseitig, so dass diese nun aneinander vorbeigedreht werden können (vgl. Figuren 4a und 4b). Indem die beiden Flügel 8a und 8b gegeneinander verdreht werden, dreht sich auch die innere Hülse 3 gegenüber der äußeren Hülse 2 (vgl. Fig. 4b). In der in Fig. 5 dargestellten zweiten Drehposition sind die beiden Hülsen 2 und 3 dabei soweit gegeneinander verdreht, dass deren Längsschnitte 6 und 7 in Umfangsrichtung fluchten, d. h. übereinander zu liegen kommen. Wie in Fig. 5b zu sehen ist, kann in dieser Position ein innerhalb der Kanüle verlaufender Katheter seitlich (d. h. in Fig. 5b nach unten) aus der Kanüle entfernt werden.

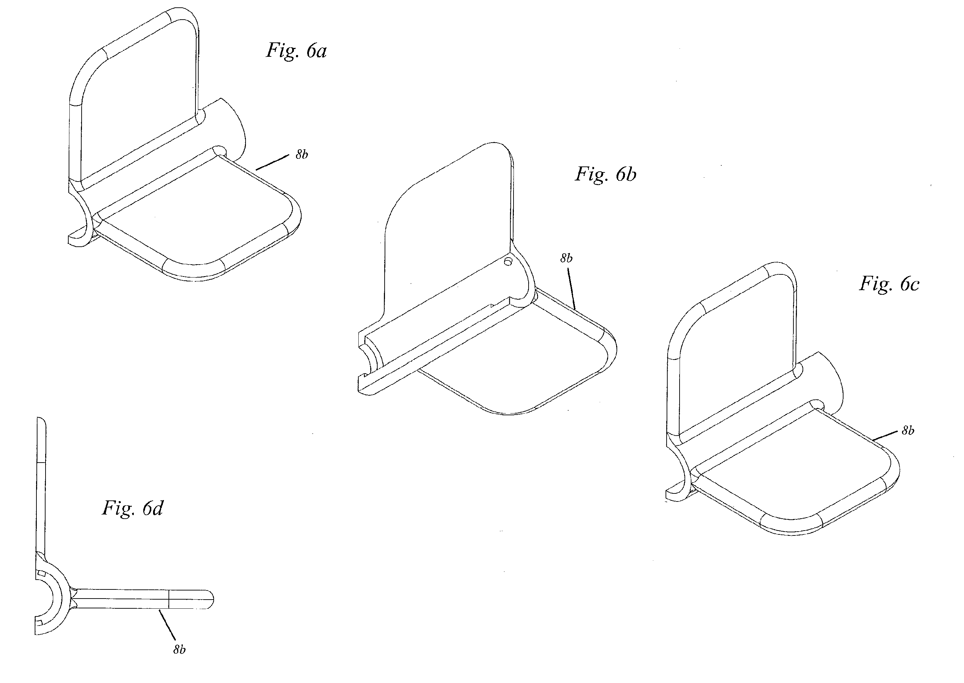

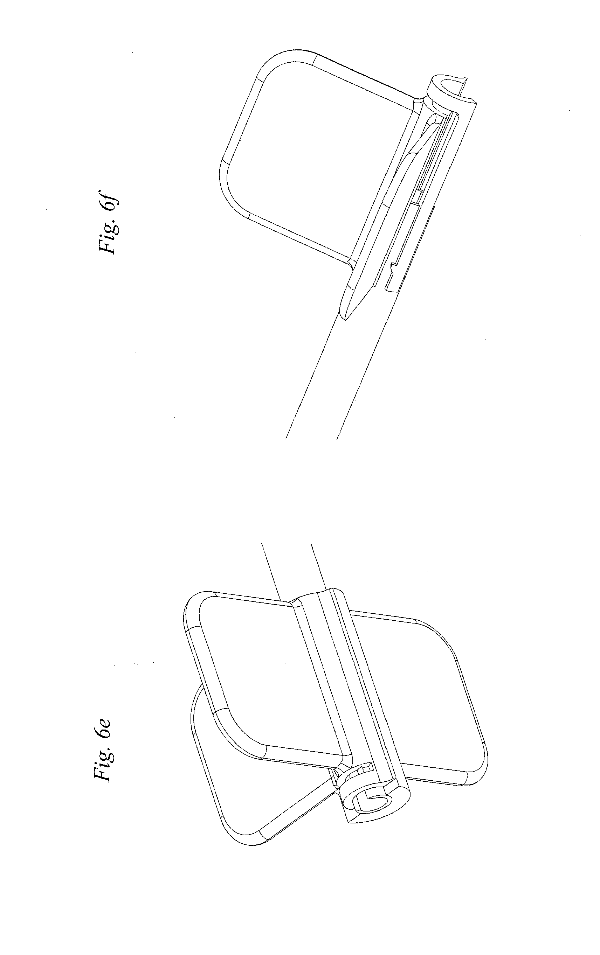

Weitere Details der Griffabschnitte bzw. Flügel sind in den Detailansichten der Figuren 6a-6f zu erkennen.

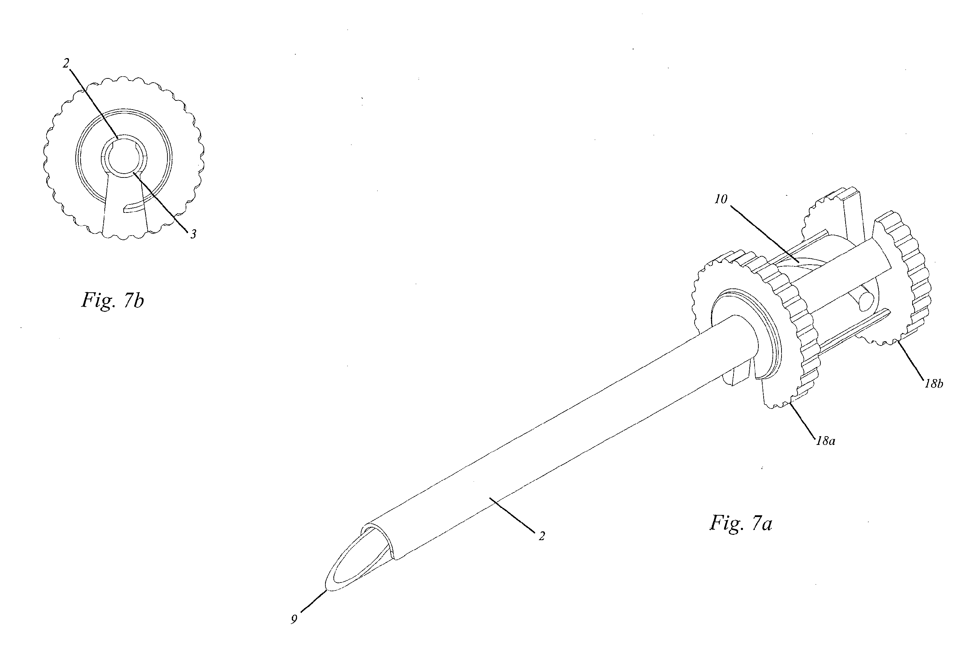

Die Figuren 7-10 zeigen die Sequenz der Figuren 1-5 für eine zweite bevorzugte Ausführungsform der erfindungsgemäßen Kanüle. Bei dieser Ausführungsform sind die beiden Flügel 8a und 8b durch zwei Griffabschnitte bzw. Drehrädchen 18a und 18b ersetzt, die über ein Gewinde 10, z. B. ein Gewinde mit großer Steigung, miteinander verbunden sind. Aufgrund dieser Gewindekonstruktion verursacht ein Verdrehen der inneren und äußeren Hülse gegeneinander gleichzeitig und automatisch ein Verschieben der Hülsen in Längsrichtung. Dadurch werden die beiden Bewegungsabschnitte, die bei der ersten Ausführungsform erforderlich sind, in einem einzigen Bewegungsabschnitt gekoppelt.

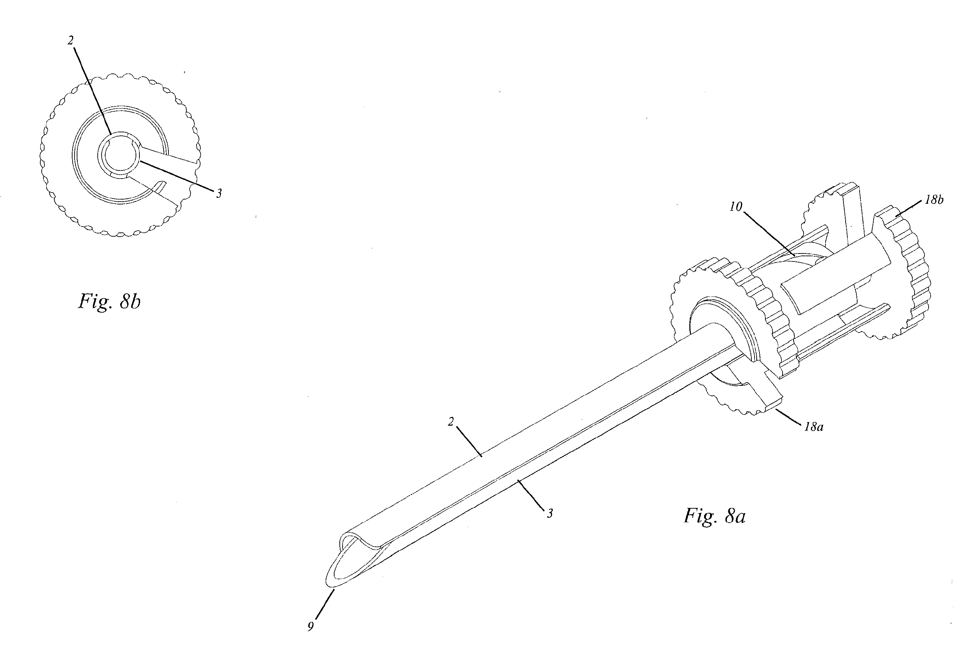

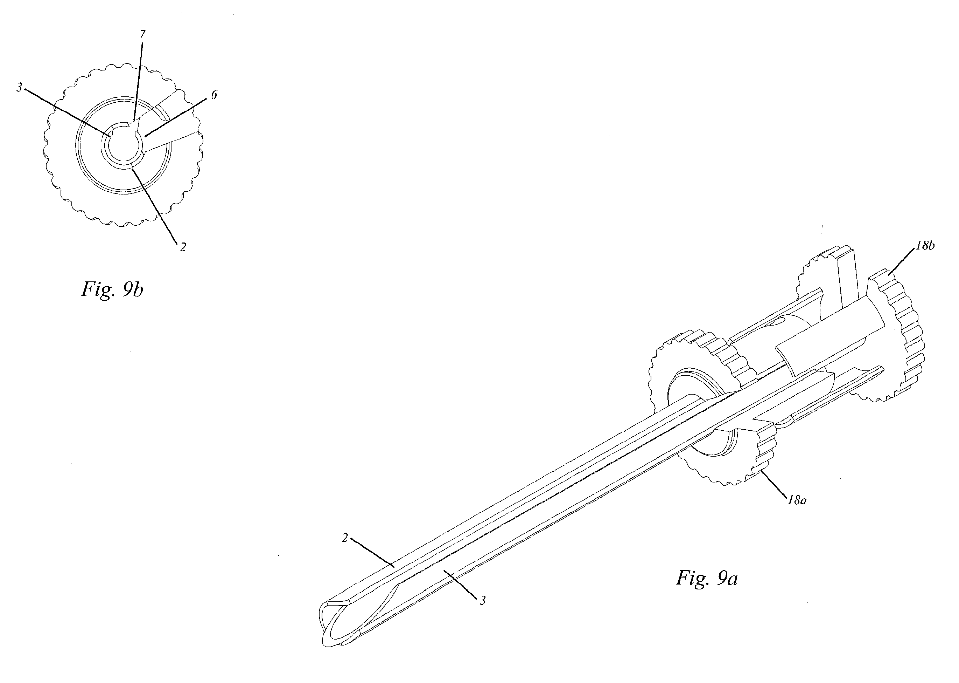

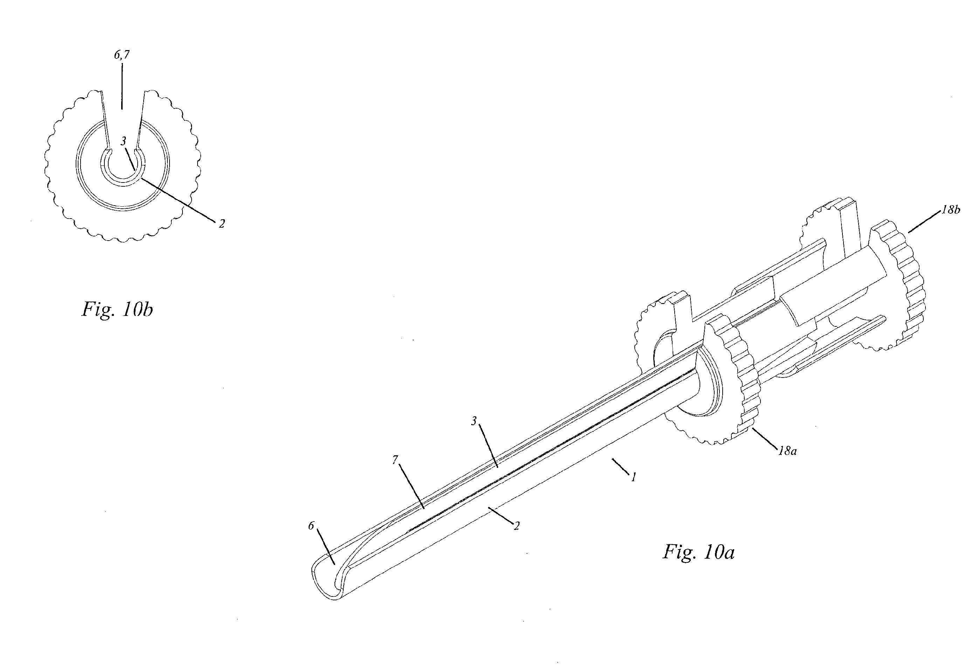

Figuren 7a und 7b zeigen die Kanüle der zweiten Ausführungsform in der ersten Drehposition, bei der der Längsschnitt 7 der inneren Hülse 3 vollständig durch die äußere Hülse 2 abgedeckt ist (vgl. Schnittansicht in Fig. 7b). Werden nun die beiden Griffabschnitte 18a und 18b gegen einander verdreht (vgl. Figuren 8 und 9), wird nicht nur die innere Hülse 3 in gleicher Weise gegenüber der äußeren Hülse 2 verdreht (vgl. Figuren 8b und 9b), sondern gleichzeitig auch die innere Hülse 3 proximal in die äußere Hülse 2 zurückgezogen (vgl. Figuren 8a und 9a). Ist die zweite Drehposition (vgl. Figuren 10a und 10b) erreicht, so dass die Längsschnitte 6 und 7 der beiden Hülsen 2 und 3 fluchten bzw. übereinander liegen, ist gleichzeitig die Punktionsspitze 9 der inneren Hülse 3 soweit in die äußere Hülse 2 zurückgezogen (vgl. Fig. 10a), dass die Punktionsspitze von der äußeren Hülse 2 abgedeckt bzw. geschützt ist. In der in Fig. 10a gezeigten zweiten Drehposition wird somit die Verletzungs- bzw. Infektionsgefahr eines Anwenders effektiv verhindert.

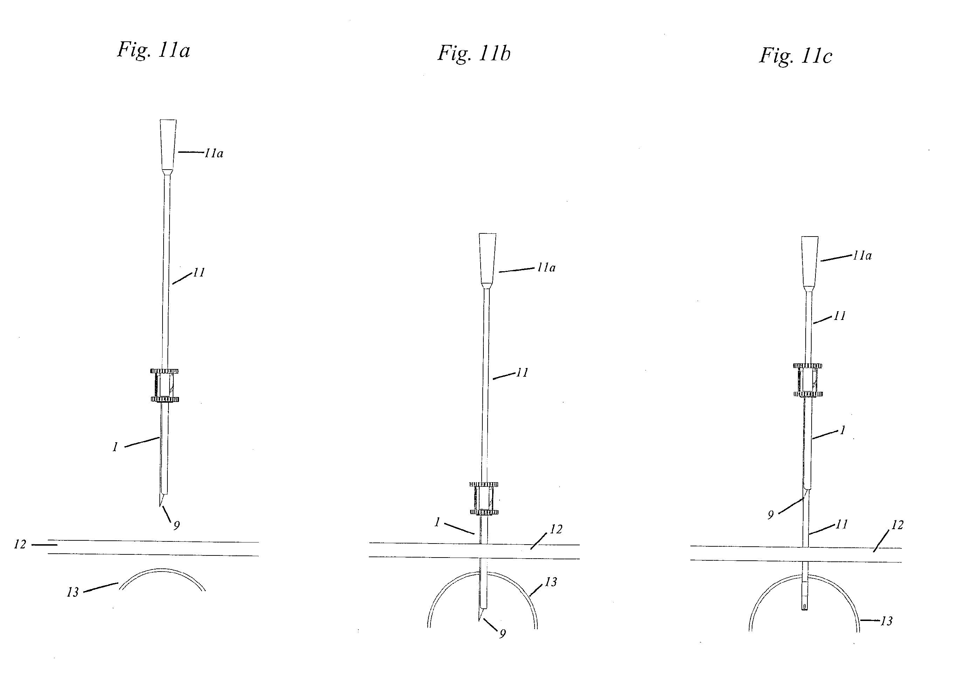

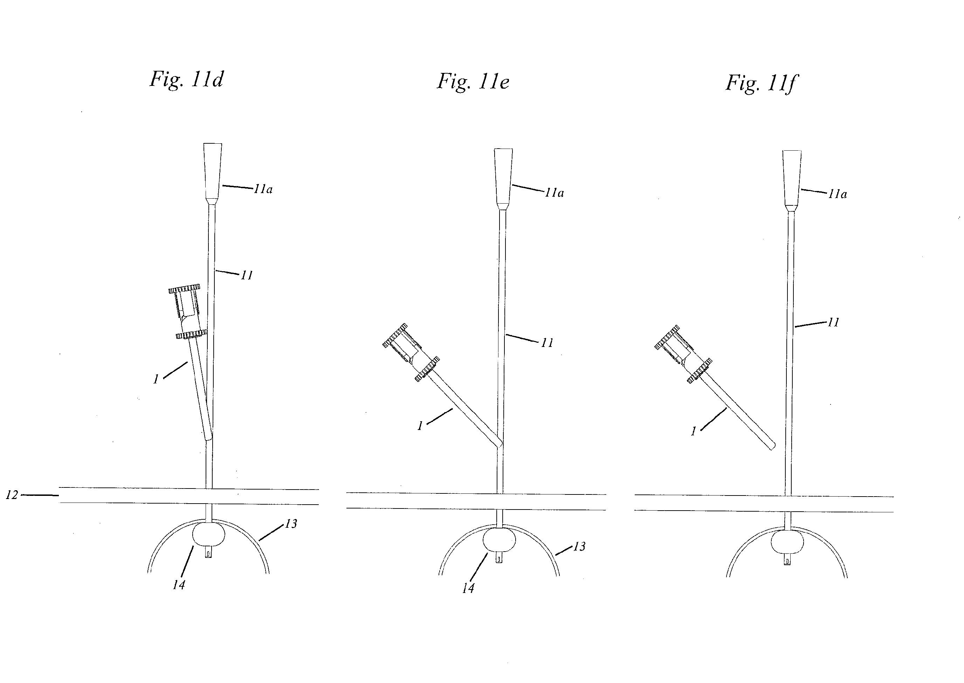

In den Figuren 11a-11f ist schematisch die Funktionsweise der erfindungsgemäßen Kanüle näher erläutert. Mit dem Bezugszeichen 13 ist ein Körperhohlraum, beispielsweise eine Blase, bezeichnet, die unterhalb einer angedeuteten Bauchdecke 12 liegt. Um den Katheter 11 in den Körperhohlraum 13 einzuführen, wird dieser in eine erfindungsgemäße Kanüle 1 eingeführt und die Kanüle 1 zusammen mit dem Katheter 11 mit Hilfe der Punktionsspitze 9 durch die Bauchdecke 12 in den Körperhohlraum 13 eingeführt (vgl. Fig. 11b). Nach dem Punktionsvorgang wird die Kanüle 1 nicht weiter benötigt, so dass diese über den Katheter 11 zurückgezogen werden kann (vgl. Fig. 11c). Allerdings verhindert der Ansatz 11a des Katheters 11 ein vollständiges Abziehen der Kanüle 1 von dem Katheter 11. Daher werden in der in Fig. 11c dargestellten Position die beiden Hülsen 2 und 3 der Kanüle 11 so weit gegeneinander verdreht, bis deren Längsschnitte übereinander zu liegen kommen, wie dies in den Figuren 10a und 10b dargestellt ist. Durch das Verdrehen der Hülsen gegeneinander wird gleichzeitig die Kanülenspitze bzw. Punktionsspitze 9 der inneren Hülse in die äußere Hülse zurückgezogen, wie oben beschrieben wurde. Nun kann der Katheter 11 seitlich durch die beiden übereinander liegenden Längsschnitte der Hülsen entfernt werden bzw. die Kanüle von dem Katheter abgenommen werden (vgl. Figuren 11d-11f). Da die Punktionsspitze 9 bei diesem Vorgang vollständig von der äußeren Hülse 2 abgedeckt bzw. geschützt wird, kann weder der Katheter 11 beschädigt werden noch der Anwender verletzt werden.

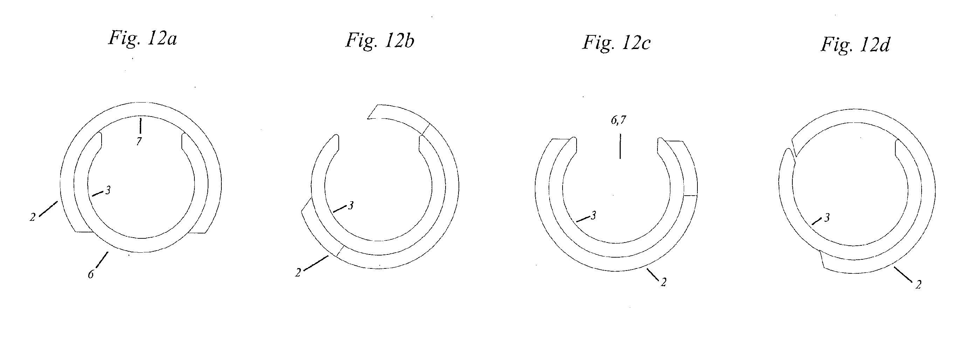

Erfindungsgemäß ist vorgesehen, dass die innere und äußere Hülse und/oder die beiden Griffabschnitte bzw. Flügel in der zweiten Position miteinander verrasten. Dadurch wird sicher gestellt, dass beim Vorgang des Entfernens der Kanüle 1 von dem Katheter 11 (vgl. Figuren 11d-11f) die Punktionsspitze 9 der inneren Hülse 3 innerhalb der äußeren Hülse 2 gesichert bleibt. Hierfür sind bevorzugt geeignete Rückhaltemittel zum Verrasten bzw. Verriegeln vorgesehen. Dies können beispielsweise Rasthaken sein, die nicht ohne weiteres lösbar sind. Dabei ist bevorzugt ein unlösbares Verrasten bzw. Verriegeln vorgesehen, so dass die Kanüle nur als Einwegkanüle verwendbar ist. Alternativ oder zusätzlich kann die Kanüle derart ausgestaltet sein, dass ein vollständiges Zurückdrehen der inneren Hülse aus der zweiten Position in die erste Position verhindert wird. Dies kann beispielsweise dadurch erzielt werden, dass die innere Hülse unter Spannung in der äußeren Hülse angeordnet ist. Wenn dann die Kanüle durch Verdrehen der beiden Hülsen gegeneinander geöffnet wird (vgl. Figuren 12a-12c), weitet sich der Durchmesser der inneren Hülse aufgrund einer Vorspannung auf. Der aufgeweitete Durchmesser der inneren Hülse blockiert dann eine Drehbewegung beim Versuch, die innere Hülse 3 wieder vollständig in die äußere Hülse 2 zu drehen, wie in Fig. 12d dargestellt.

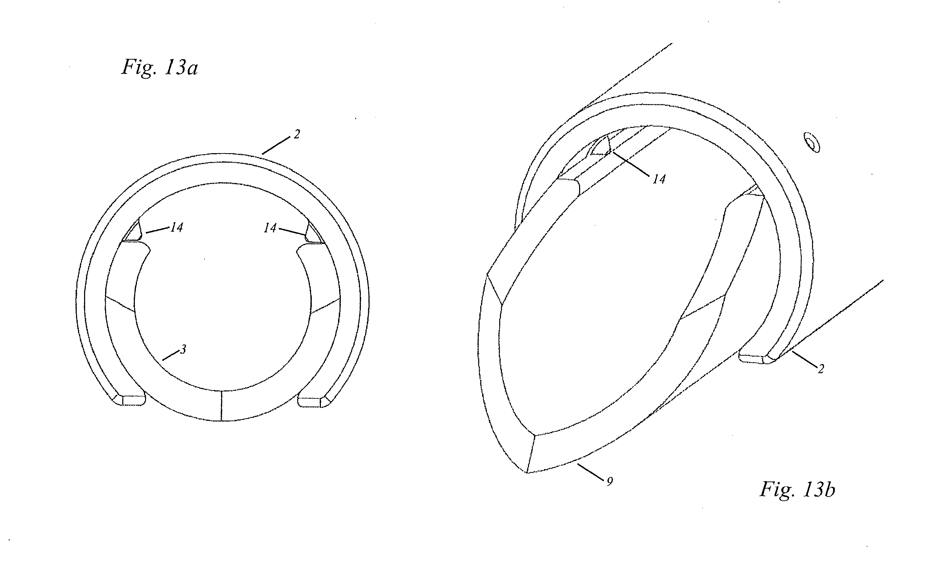

Erfindungsgemäß ist die scharfe Punktionsspitze nur an der inneren Hülse vorgesehen, wohingegen das distale Ende der äußeren Hülse stumpf und/oder derart ausgebildet ist, dass eine Verletzung an diesem distalen Ende weitestgehend vermieden wird. Dies bedeutet, dass die Punktion lediglich mit Hilfe der inneren Hülse, die den Schliff aufweist, erfolgt. Mit anderen Worten wird die für die Punktion erforderliche Kraft von der Punktionsspitze der inneren Hülse auf das Gewebe übertragen. Hierfür ist eine stabile Lagerung der inneren Hülse von Vorteil. Es ist daher erfindungsgemäß bevorzugt, dass die Innenfläche der äußeren Hülse an ihrem distalen Ende zwei Stabilisierungselemente aufweist, die die innere Hülse bei der Punktion stabilisieren. Beispielsweise kann eine solche Stabilisierung mit Hilfe zweiter Vorsprünge oder Überstände 14, wie in den Figuren 13a und 13b dargestellt, erzielt werden. Diese beiden Vorsprünge 14, die an der Innenfläche der äußeren Hülse vorgesehen sind, dienen der inneren Hülse als Anlagepunkte während der Punktion. Dadurch wird die innere Kanüle gegenüber Druck- und Torsionskräften, die bei der Punktion auftreten können, stabilisiert. So wird ein Zurückfedern der inneren Hülse innerhalb der äußeren Hülse verhindert, was das Punktionsgefühl für den Anwender verbessert.

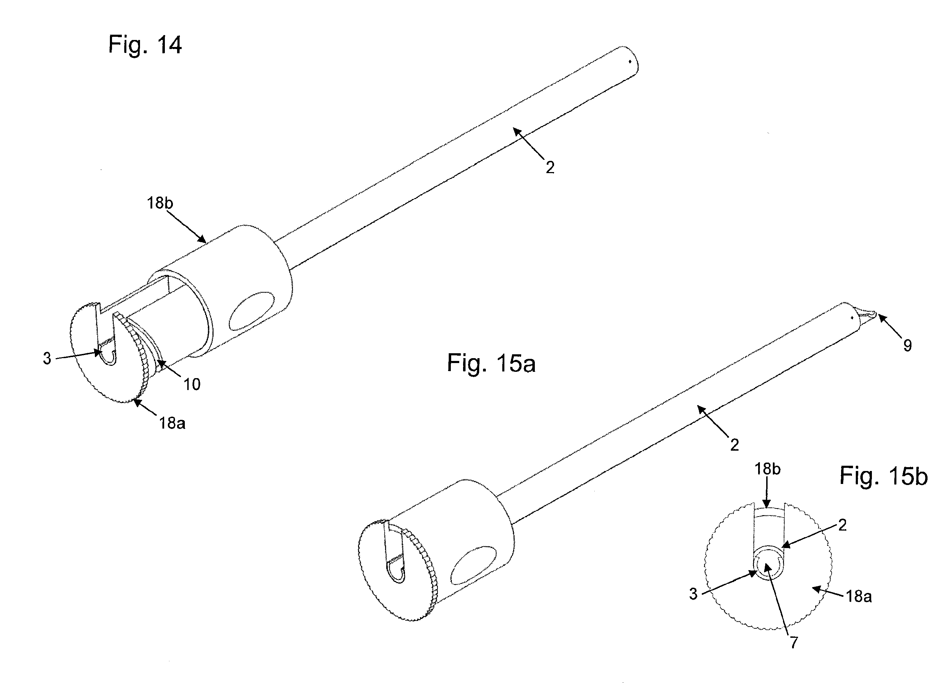

Fig. 14 zeigt eine erfindungsgemäße Kanüle gemäß einer dritten Ausführungsform mit zwei Griffabschnitten 18a und 18b, die über ein Gewinde 10, z. B. ein Gewinde mit großer Steigung, miteinander verbunden sind. Aufgrund dieser Gewindekonstruktion verursacht ein Verdrehen der inneren und äußeren Hülse gegeneinander wie bei der zweiten Ausführungsform gleichzeitig und automatisch ein Verschieben der Hülsen in Längsrichtung.

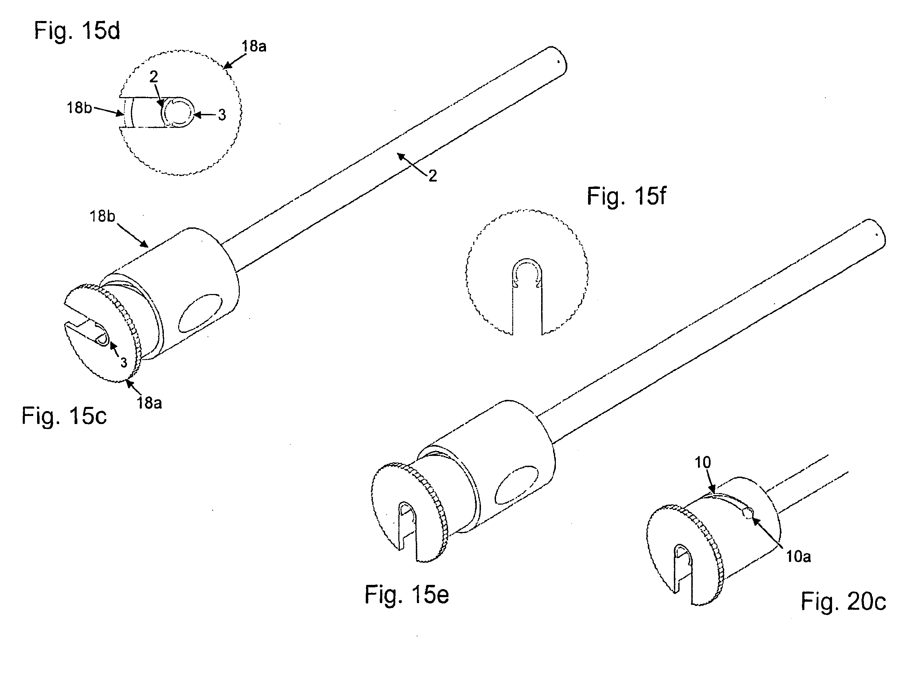

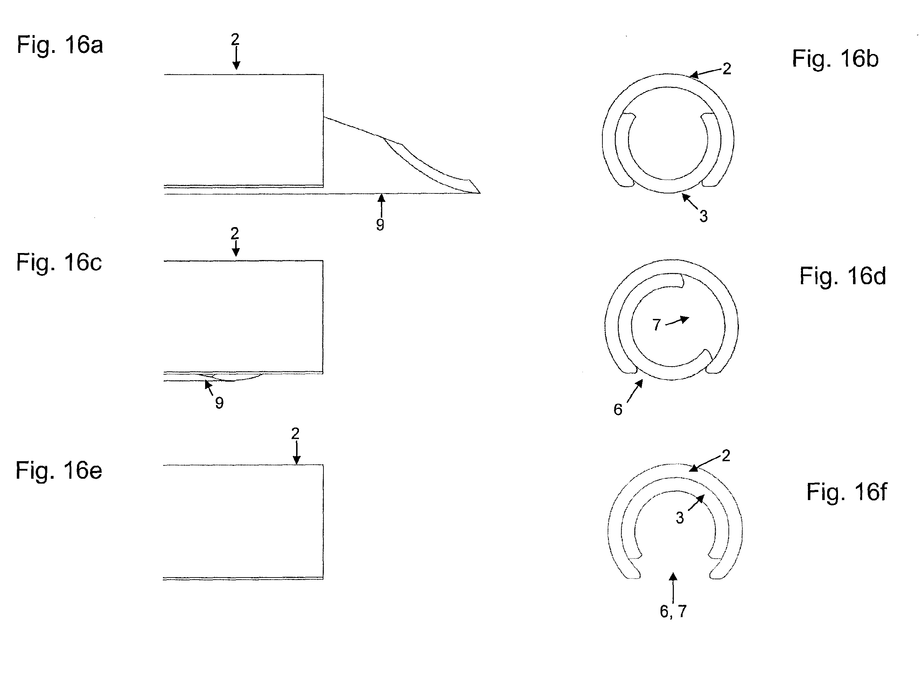

Figuren 15a und 15b zeigen die Kanüle der dritten Ausführungsform in der ersten Drehposition, bei der der Längsschnitt 7 der inneren Hülse 3 vollständig durch die äußere Hülse 2 abgedeckt ist (vgl. Schnittansicht in Fig. 15b und 16b). In dieser Drehposition lässt sich beispielsweise eine Punktion durchführen. Werden nun die beiden Griffabschnitte 18a und 18b gegen einander verdreht (vgl. Figuren 15c und 15e), wird nicht nur die innere Hülse 3 in gleicher Weise gegenüber der äußeren Hülse 2 verdreht (vgl. Figuren 15d, 15f, 16d und 16f), sondern gleichzeitig auch die innere Hülse 3 proximal in die äußere Hülse 2 zurückgezogen (vgl. Figuren 16a, 16c und 16e). Bereits in der in den Figuren 15c, 15d, 16c und 16d dargestellten dritten Drehposition ist die Punktionsspitze 9 der inneren Hülse 3 soweit in die äußere Hülse 2 zurückgezogen (vgl. Fig. 16c), dass die Punktionsspitze von der äußeren Hülse 2 abgedeckt bzw. geschützt ist. In der in Fig. 15c gezeigten dritten Drehposition wird somit die Verletzungs- bzw. Infektionsgefahr eines Anwenders effektiv verhindert. Ist die zweite Drehposition (vgl. Figuren 15e, 15f, 16e und 16f) erreicht, so liegen die Längsschnitte 6 und 7 der beiden Hülsen 2 und 3 übereinander bzw. fluchten.

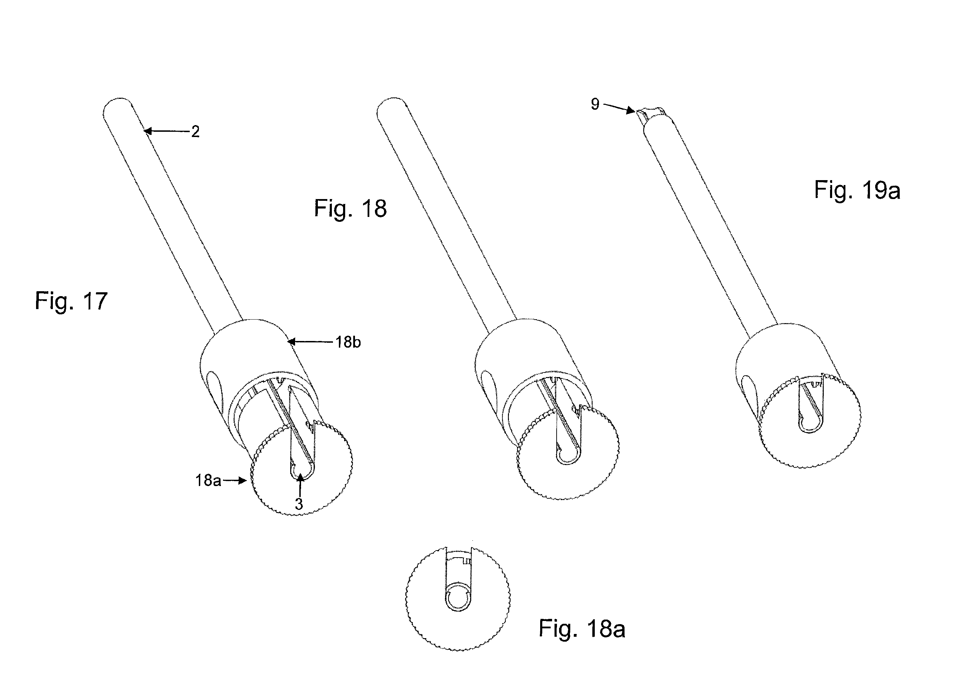

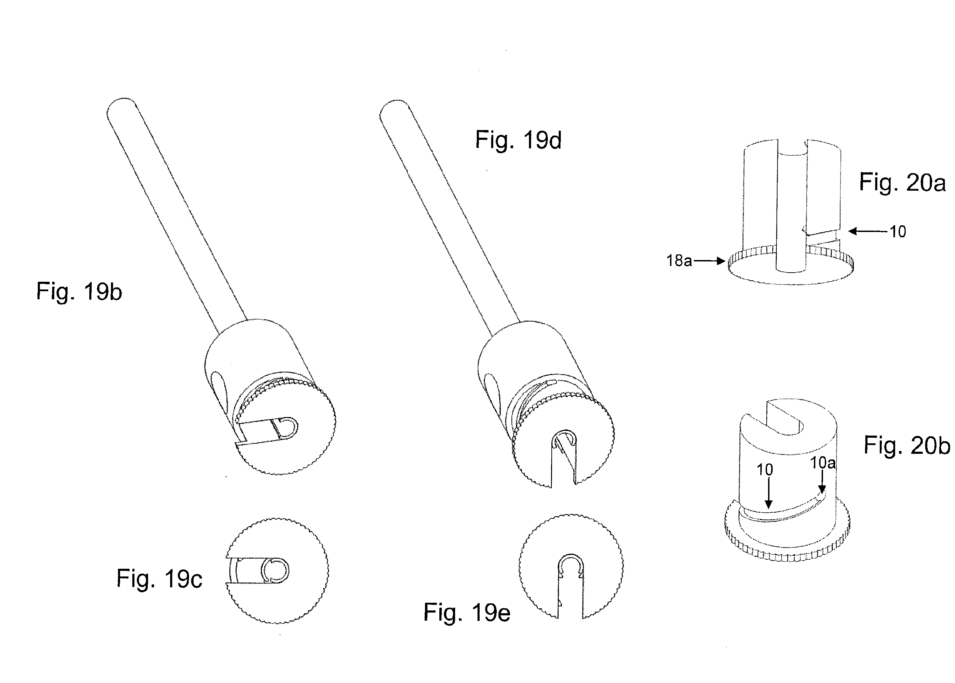

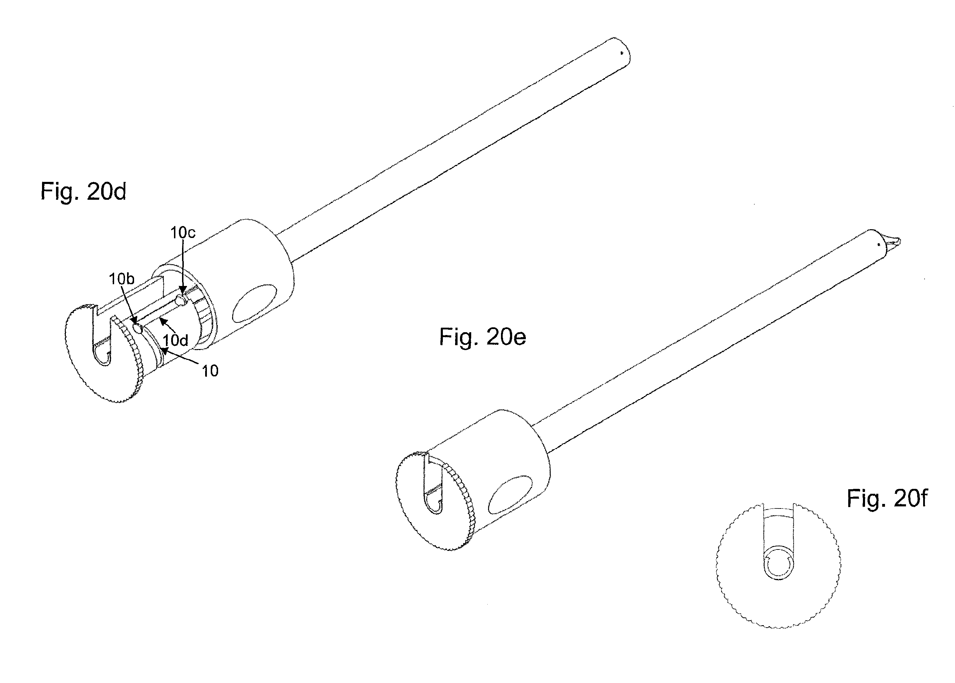

Fig. 17 zeigt die Kanüle gemäß der dritten Ausführungsform vor deren Montage, so dass erkennbar wird, wie die beiden Griffabschnitte 18a und 18b ineinanderpassen und zusammenwirken. Die Figuren 18 und 18a zeigen die Kanüle nach der Montage, aber vor ihrem Einsatz, d.h. der Griffabschnitt 18a ist soweit in den Griffabschnitt 18b eingeführt, dass beide Abschnitte ineinander eingreifen. Die Kanülenspitze 9 ist dabei innerhalb der äußeren Hülse 2 geschützt gelagert. Erst in der Punktionsposition, die in Fig. 19a dargestellt ist, tritt die Kanülenspitze zum Punktieren nach außen. Das Ineinandergreifen der beiden Griffabschnitte lässt sich beispielsweise dadurch erreichen, dass der erste Griffabschnitt 18a eine Rastöffnung 10c (vgl. Fig. 20d) aufweist, in die eine nicht dargestellte Nase im Inneren des zweiten Griffabschnittes 18b einrastet. Dadurch wird die Kanüle in der in Fig. 18 dargestellten Situation fixiert bzw. gesichert.

Um die Kanüle aus der in Fig. 18 dargestellten Montageposition in die in Fig. 19a gezeigte Punktionsposition zu überführen, muss der Anwender den ersten Griffabschnitt 18a in distaler Richtung in den zweiten Griffabschnitt 18b hineinschieben bzw. -drücken. Dabei löst sich die Nase aus der Rastöffnung 10c und gleitet durch die Nut 10d in proximaler Richtung, bis sie in der Rastöffnung 10b irreversibel einrastet, da die Rastöffnung 10b tiefer ausgebildet ist als die Vertiefung der Nut 10d. Somit wird die Kanülenspitze wie in Fig. 19a dargestellt sicher fixiert, so dass sie beim Punktieren nicht in die Hülse 2 zurückgleiten kann.

Wenn die innere Hülse 3 zusammen mit der Kanülenspitze 9 in die äußere Hülse 2 durch Verdrehen der beiden Griffabschnitte 18a und 18b gegeneinander zurückgezogen wird (vgl. Fig. 19b-19d), gleitet die Nase durch das Gewinde 10 (vgl. Fig. 20d und 20b) bis zu einer weiteren Rastöffnung 10a (vgl. Fig. 20b), in die die Nase einrastet und somit die Kanüle in der zweiten Drehposition fixiert bzw. gesichert wird. Nach Bedarf können weitere Rastpositionen vorgesehen sein; es ist aber bevorzugt, dass die Griffabschnitte und/oder die Hülsen zumindest in der ersten und zweiten Drehposition miteinander verrasten.

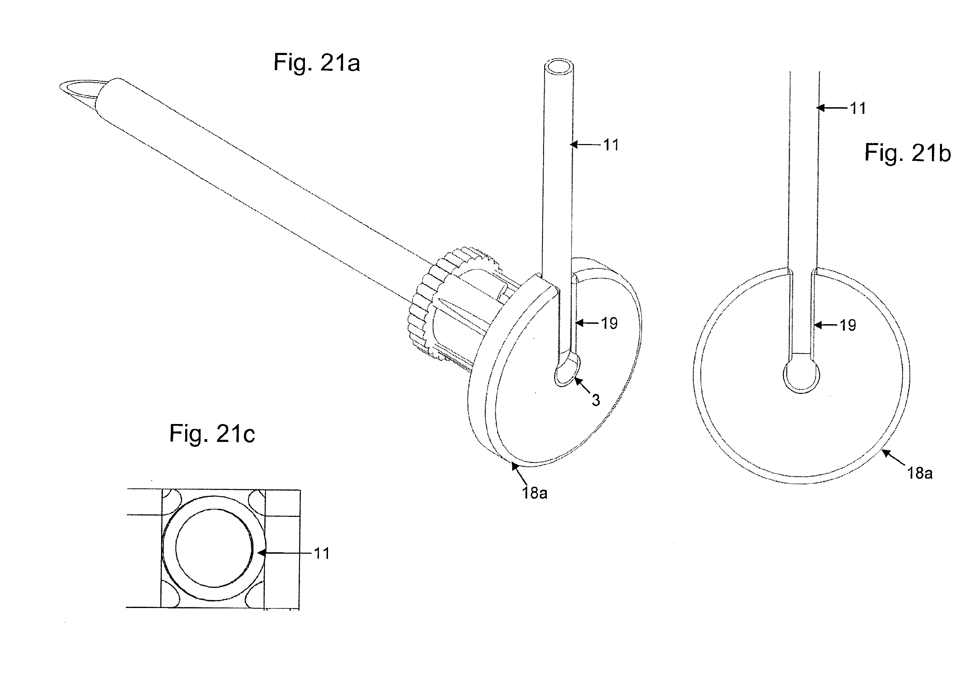

Die Figuren 21a-21c zeigen eine erfindungsgemäße Kanüle gemäß einer vierten Ausführungsform. Bei dieser Ausführungsform ist der mit der inneren Hülse 3 verbundene Griffabschnitt 18a als Druckplatte ausgebildet. Diese dient dazu, dass der Anwender z.B. mittels der Handfläche oder des Daumens beim Punktieren flächig Druck ausüben kann. Dies erleichtert dem Anwender das Punktieren und ermöglicht somit eine sicherere Arbeitsweise. Da gegebenenfalls die innere Hülse 3 einen Katheter 11 aufnehmen können soll, weist die Druckplatte bevorzugt einen Schlitz bzw. eine Führung 19 für einen Katheter 11 auf, damit verhindert werden kann, dass der Katheter z.B. während des Punktierens zwischen Druckplatte und Handfläche oder Daumen eingeklemmt oder gequetscht werden kann. Der Katheter 11 wird, wie in Fig. 21c gezeigt, z.B. mittels mehrerer Nasen in der Führung 19 plan in der Ebene der Druckplatte 18a zu deren Mittelpunkt geführt, von wo aus sich der Katheter 11 in die innere Hülse 3 hinein erstreckt.

Die äußere Hülse 2 und/oder die Griffabschnitte 18a und/oder 18b sind bevorzugt aus Kunststoff gefertigt. Geeignete Kunststoffe umfassen unter anderem Polyamide, Polysulfone und Hochleistungskunststoffe wie z.B. Polyetheretherketon (PEEK). Eine äußere Hülse aus Kunststoff stellt einen besonders wirksamen Schutz für die zurückgezogene Kanülenspitze dar, da eine solche Kunststoffhülse nur schwer bricht und an ihrem distalen Ende keine scharfen Kanten ausbildet, die ebenfalls zu Verletzungen führen könnten.

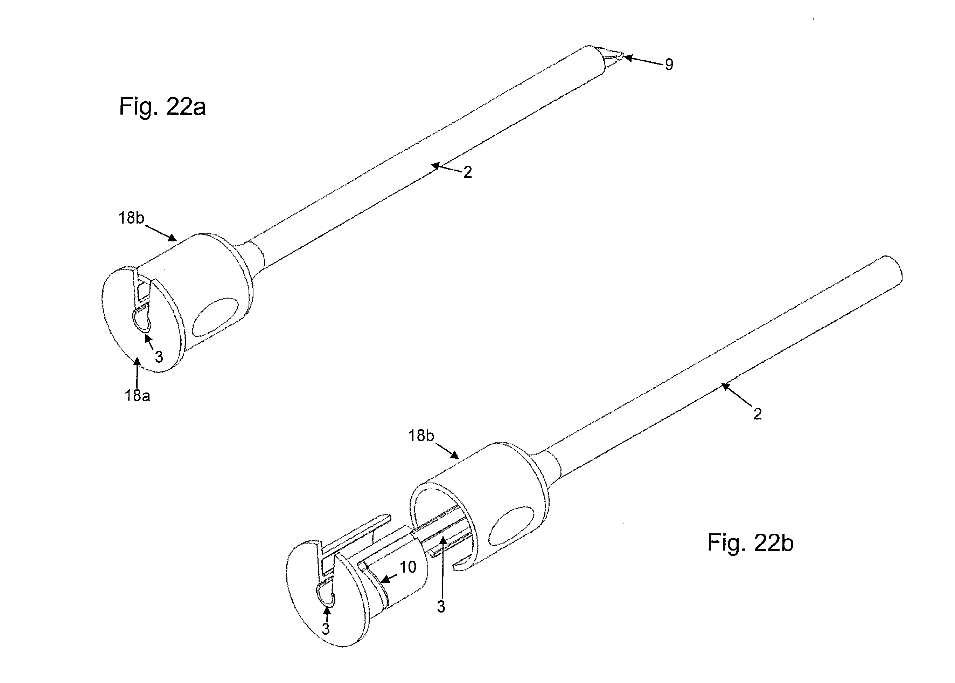

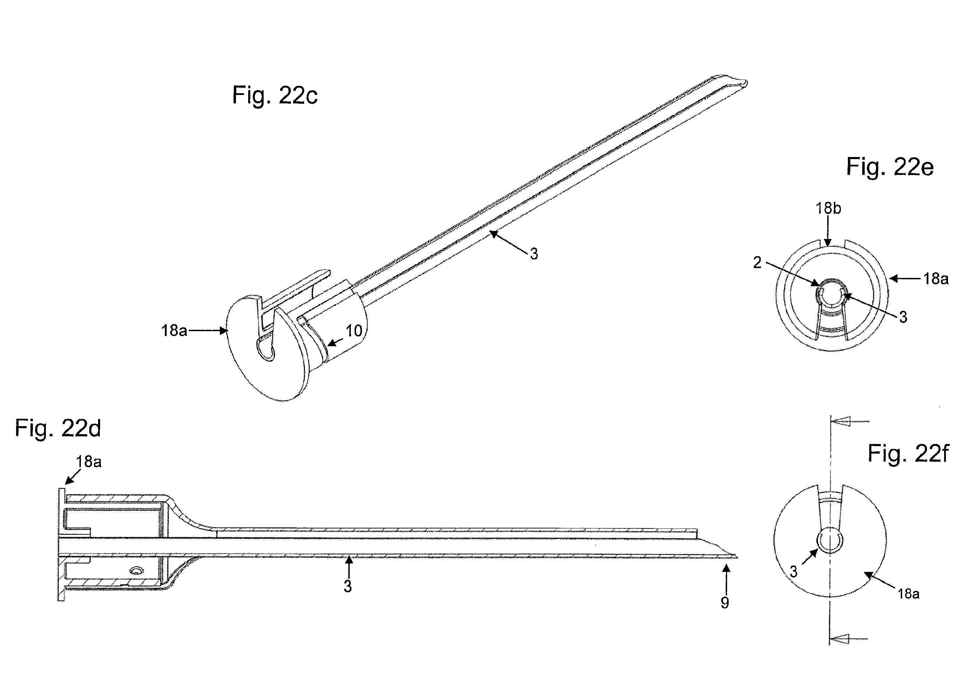

Besonders bevorzugt sind dabei die äußere Hülse 2 und der damit verbundene Griffabschnitt 18b einstückig bzw. einteilig aus Kunststoff hergestellt. Hierdurch wird eine einfache und damit kostengünstige Herstellung ermöglicht. Eine solche Ausführungsform ist beispielhaft in den Figuren 22a-22f zu sehen. Die äußere Hülse 2 und der damit verbundene Griffabschnitt 18b sind dabei derart geformt, dass sie auf einfache und kostengünstige Weise aus Kunststoff (spritz-)gegossen werden können. Auf diese Weise lässt sich auch die nicht erkennbare Nase im Inneren des Griffabschnittes 18b ohne Probleme fertigen

Die erfindungsgemäße Sicherheitskanüle verringert das Verletzungs- und Infektionsrisiko bei der Verwendung gegenüber herkömmlichen Punktionskanülen, insbesondere Spaltkanülen. Die Sicherheitskanüle gemäß der vorliegenden Anmeldung ist kostengünstig herstellbar und einfach anwendbar. Der Sicherheitsmechanismus funktioniert automatisch und erfordert keine spezielle Schulung des Personals. Ferner ist das Konzept der vorliegenden Sicherheitskanüle variabel einsetzbar und lässt sich insbesondere auf unterschiedliche Punktionskanülen und verschiedene Katheter anwenden.

## Patentansprüche

1. Suprapubische Kanüle (1) zur Punktion von Körperhohlräumen mit einer äußeren und einer inneren längsgeschnittenen Hülse (2, 3) mit proximalen und distalen Enden (4, 5) und zwei Griffabschnitten (8a, 8b; 18a, 18b) am proximalen Ende (5), wobei die innere Hülse (3) innerhalb der äußeren Hülse (2) in Längsrichtung verschiebbar angeordnet und in der äußeren Hülse (2) derart drehbar ist, dass der Längsschnitt (7) der inneren Hülse in einer ersten Drehposition durch die äußere Hülse (2) abgedeckt wird und die Längsschnitte (6, 7) von innerer und äußerer Hülse in einer zweiten Drehposition übereinander zu liegen kommen, so dass ein Katheter durch die Längsschnitte aus der inneren Hülse entfernt werden kann, wobei die innere Hülse (3) an ihrem distalen Ende (4) eine Punktionsspitze (9) aufweist, die vollständig in die äußere Hülse (2) zurückgezogen ist, wenn sich die innere Hülse (3) in der zweiten Drehposition befindet, **dadurch gekennzeichnet, dass** die innere und äußere Hülse (2, 3) und/oder die beiden Griffabschnitte (8a, 8b; 18a, 18b) in der zweiten Drehposition miteinander verrasten, so dass die Punktionsspitze innerhalb der äußeren Hülse gesichert bleibt

2. Kanüle nach Anspruch 1, wobei die innere und äußere Hülse (2, 3) und/oder die beiden Griffabschnitte (8a, 8b; 18a, 18b) in der zweiten Position unlösbar miteinander verrasten

3. Kanüle nach Anspruch 1 oder 2, wobei die innere Hülse (3) nur dann in die zweite Position gedreht werden kann, wenn die Punktionsspitze (9) der inneren Hülse (3) bereits vollständig in die äußere Hülse (2) zurückgezogen ist.

4. Kanüle nach einem der vorigen Ansprüche, wobei die zwei Griffabschnitte (8a, 8b; 18a, 18b) eine Drehung der inneren Hülse (3) blockieren, solange sich die Punktionsspitze (9) der inneren Hülse distal aus der äußeren Hülse (2) heraus erstreckt.

5. Kanüle nach einem der vorigen Ansprüche, wobei ein erster Griffabschnitt (8a; 18a) mit der inneren Hülse (3) verbunden ist und ein zweiter Griffabschnitt (8b; 18b) mit der äußeren Hülse (2) verbunden ist.

6. Kanüle nach Anspruch 1, wobei eine Drehung der inneren Hülse (3) in die zweite Position bewirkt, dass die Punktionsspitze (9) der inneren Hülse (3) vollständig in die äußere Hülse (2) zurückziehbar wird und wobei bevorzugt am proximalen Ende der Hülsen jeweils Bereiche mit einem vergrößerten Durchmesser vorgesehen sind und die Dreh- und die Verschiebebewegung der Hülsen mittels eines Gewindes (10) an diesen Bereichen mit einem vergrößerten Durchmesser miteinander gekoppelt sind.

7. Kanüle nach Anspruch 6, wobei die Kanüle (1) an ihrem proximalen Ende (5) einen ersten, mit der inneren Hülse (3) verbundenen Griffabschnitt (18a) und einen zweiten, mit der äußeren Hülse (2) verbundenen Griffabschnitt (18b) aufweist und wobei eine relative Drehbewegung der Griffabschnitte bewirkt, dass die Punktionsspitze (9) der inneren Hülse vollständig in die äußere Hülse (2) zurückgezogen wird und die Längsschnitte (6, 7) von innerer und äußerer Hülse übereinander zu liegen kommen.

8. Kanüle nach einem der vorigen Ansprüche, wobei das distale Ende (4) der äußeren Hülse im wesentlichen stumpf ist.

9. Kanüle nach einem der vorigen Ansprüche, die derart ausgebildet ist, dass ein vollständiges Zurückdrehen der inneren Hülse (3) aus der zweiten Position in die erste Position verhindert wird und wobei sich die innere Hülse (3) bevorzugt in der zweiten Position aufweitet, was ein Zurückdrehen in die erste Position unmöglich macht.

10. Kanüle nach einem der vorigen Ansprüche, wobei die Innenfläche der äußeren Hülse (2) an ihrem distalen Ende (4) zwei Stabilisierungselemente (14) aufweist, die die innere Hülse (3) bei der Punktion stabilisieren.

11. Kanüle nach einem der vorigen Ansprüche, wobei zwischen der ersten und zweiten Drehposition eine dritte Drehposition vorgesehen ist, in der die Punktionsspitze (9) bereits vollständig in die äußere Hülse (3) zurückziehbar ist, die Längsschnitte (6, 7) von innerer und äußerer Hülse aber noch nicht übereinander zu liegen kommen, wobei die Kanüle bevorzugt derart ausgebildet ist, dass ein vollständiges Zurückdrehen der inneren Hülse (3) aus der dritten Position in die erste Position verhindert wird und/oder wobei eine Drehung der inneren Hülse (3) in die dritte Position bevorzugt bewirkt, dass die Punktionsspitze (9) der inneren Hülse (3) vollständig in die äußere Hülse (2) zurückziehbar wird.

12. Kanüle nach Anspruch 11, wobei die Kanüle (1) an ihrem proximalen Ende (5) einen ersten, mit der inneren Hülse (3) verbundenen Griffabschnitt (18a) und einen zweiten, mit der äußeren Hülse (2) verbundenen Griffabschnitt (18b) aufweist und wobei eine relative Drehbewegung der Griffabschnitte bewirkt, dass die Punktionsspitze (9) der inneren Hülse in der dritten Drehposition vollständig in die äußere Hülse (2) zurückgezogen wird und die Längsschnitte (6, 7) von innerer und äußerer Hülse in der zweiten Drehposition übereinander zu liegen kommen und wobei bevorzugt an den Griffabschnitten (18a, 18b) ein Gewinde (10) vorgesehen ist, durch das die Dreh- und die Verschiebebewegung der Hülsen miteinander gekoppelt sind.

13. Kanüle nach Anspruch 7 oder 12, wobei die äußere Hülse (2) und der zweite Griffabschnitt (18b) einteilig aus Kunststoff hergestellt sind.

14. Kanüle nach einem der Ansprüche 5, 7, 12 und 13, wobei der mit der inneren Hülse (3) verbundene Griffabschnitt (8a; 18a) eine Druckplatte aufweist.

15. Kit mit einer Kanüle (1) gemäß einem der vorigen Ansprüche und einem Katheter (11), der in der inneren Hülse (3) der Kanüle (1) aufnehmbar ist, wobei der Katheter (11) in der zweiten Kanülenposition durch die übereinanderliegenden Längsschnitte (6, 7) der Hülsen (2, 3) aus der Kanüle (1) entfernbar ist.

## Claims

1. A suprapubic cannula (1) for puncturing body cavities comprising an outer and an inner longitudinally cut sleeve (2, 3) having proximal and distal ends (4, 5) and two handle portions (8a, 8b; 18a, 18b) at its proximal end (5), wherein the inner sleeve (3) is arranged within the outer sleeve (2) to be longitudinally displaceable and rotatable within the outer sleeve (2) such that the longitudinal cut (7) of the inner sleeve is covered in a first rotational position by the outer sleeve (2) and the longitudinal cuts (6, 7) of the inner and the outer sleeve are placed on top of each other in a second rotational position such that a catheter can be removed from the inner sleeve via the longitudinal cuts, wherein the inner sleeve (3) is provided at its distal end (4) with a puncture tip (9) that is completely retracted into the outer sleeve (2) if the inner sleeve (3) is in the second rotational position, **characterized in that** the inner and outer sleeves (2, 3) and/or the two handle portions (8a, 8b; 18a, 18b) interlock in the second rotational position such that the puncture tip is secured within the outer sleeve.

2. The cannula of claim 1, wherein the inner and the outer sleeve (2, 3) and/or the two handle portions (8a, 8b; 18a, 18b) are non-detachably interlocked with each other in the second position.

3. The cannula of claim 1 or 2, wherein the inner sleeve (3) can only be rotated into the second position if the puncture tip (9) of the inner sleeve (3) has already been completely retracted into the outer sleeve (2).

4. The cannula of any of the preceding claims, wherein the two handle portions (8a, 8b; 18a, 18b) prevent the inner sleeve (3) from rotating as long as the puncture tip (9) of the inner sleeve distally extends from the outer sleeve (2).

5. The cannula of any of the preceding claims, wherein a first handle portion (8a; 18a) is connected to the inner sleeve (3) and a second handle portion (8b; 18b) is connected to the outer sleeve (2).

6. The cannula of claim 1, wherein upon rotation of the inner sleeve (3) into the second position the puncture tip (9) of the inner sleeve (3) is completely retractable into the outer sleeve (2), wherein each proximal end of the sleeves has areas with a widened diameter and wherein the rotational and the shifting movement of the sleeves are coupled by means of a thread (10) at these areas with a widened diameter.

7. The cannula of claim 6, wherein the proximal end (5) of the cannula (1) is provided with a first handle portion (18a) connected to the inner sleeve (3) and a second handle portion (18b) connected to the outer sleeve (2) and wherein upon a rotational movement of the handle portions relative to each other the puncture tip (9) of the inner sleeve is completely retracted into the outer sleeve (2) and the longitudinal cuts (6, 7) of the inner and outer sleeves are placed on top of each other.

8. The cannula of any of the preceding claims, wherein the distal end (4) of the outer sleeve is essentially blunt.

9. The cannula of any of the preceding claims, configured such that the inner sleeve (3) is prevented from completely turning from the second position back into the first position, and wherein the inner sleeve (3) expands preferably in the second position so as to prevent it from turning back into the first position.

10. The cannula of any of the preceding claims, wherein the distal end (4) of the inner surface of the outer sleeve (2) is provided with two stabilising elements (14) which stabilise the inner sleeve (3) during puncturing.

11. The cannula of any of the preceding claims, wherein between the first and second rotational positions a third rotational position is provided in which the puncture tip (9) is already completely retractable into the outer sleeve (3), while the longitudinal cuts (6, 7) of the inner and outer sleeves are not yet placed on top of each other, and wherein the cannula is configured such that the inner sleeve (3) is prevented from completely turning from the third position back into the first position, and/or wherein upon rotation of the inner sleeve (3) into the third position the puncture tip (9) of the inner sleeve (3) preferably is completely retractable into the outer sleeve (2).

12. The cannula of claim 11, wherein the proximal end (5) of the cannula (1) is provided with a first handle portion (18a) connected to the inner sleeve (3) and a second handle portion (18b) connected to the outer sleeve (2) and wherein upon a rotational movement of the handle portions relative to each other the puncture tip (9) of the inner sleeve is completely retracted into the outer sleeve (2) in the third rotational position and the longitudinal cuts (6, 7) of the inner and outer sleeves are placed on top of each other in the second rotational position, and wherein the handle portions (18a, 18b) preferably are provided with a thread (10) that couples the rotational and shifting movements of the sleeves with one another.

13. The cannula of claim 7 or 12, wherein the outer sleeve (2) and the second handle portion (18b) are made of one plastics piece.

14. The cannula of any of claims 5, 7, 12 and 13, wherein the handle portion (8a; 18a) connected to the inner sleeve (3) is provided with a pressure plate.

15. A kit comprising a cannula (1) according to any of the preceding claims and a catheter (11) that is receivable in the inner sleeve (3) of the cannula (1), wherein the catheter (11) is removable from the cannula (1) in the second position of the cannula via the longitudinal cuts (6, 7) of the sleeves (2, 3) placed on top of each other.

## Revendications

1. Canule suprabienne (1) pour la ponction de cavités corporelles, comprenant une chemise externe et une chemise interne fendue en longueur (2, 3) avec une extrémité proximale et une extrémité distale (4, 5) et deux sections de préhension (8a, 8b ; 18a, 18b) à l'extrémité proximale (5), la chemise interne (3) étant disposée de manière à pouvoir coulisser dans le sens de la longueur à l'intérieur de la chemise externe (2) et à pouvoir tourner dans la chemise externe (2) de telle manière que la fente longitudinale (7) de la chemise interne est recouverte par la chemise externe (2) dans une première position de rotation et que les fentes longitudinales (6, 7) de la chemise interne et de la chemise externe viennent à se superposer dans une deuxième position de rotation, si bien qu'un cathéter peut être retiré de la chemise interne par les fentes longitudinales, la chemise interne (3) présentant une aiguille de ponction (9) à son extrémité distale (4), laquelle est entièrement rétractée dans la chemise externe (2) quand la chemise interne (3) se trouve en deuxième position de rotation, **caractérisée en ce que** la chemise interne et la chemise externe (2, 3) et/ou les deux sections de préhension (8a, 8b ; 18a, 18b) s'enclenchent l'une avec l'autre en deuxième position de rotation, de telle manière que l'aiguille de ponction reste fixée à l'intérieur de la chemise externe.

2. Canule selon la revendication 1, où la chemise interne et la chemise externe (2, 3) et/ou les deux sections de préhension (8a, 8b ; 18a, 18b) s'enclenchent l'une avec l'autre de manière inamovible en deuxième position.

3. Canule selon la revendication 1 ou la revendication 2, où la chemise interne (3) ne peut être tournée en deuxième position que si l'aiguille de ponction (9) de la chemise interne (3) est déjà entièrement rétractée dans la chemise externe (2).

4. Canule selon l'une des revendications précédentes, où les deux sections de préhension (8a, 8b ; 18a, 18b) bloquent une rotation de la chemise interne (3) tant que l'aiguille de ponction (9) de la chemise interne s'étend distalement hors de la chemise externe (2).

5. Canule selon l'une des revendications précédentes, où une première section de préhension (8a ; 18a) est raccordée à la chemise interne (3) et une deuxième section de préhension (8b ; 18b) est raccordée à la chemise externe (2).

6. Canule selon la revendication 1, où une rotation de la chemise interne (3) en deuxième position entraîne la rétractabilité complète de l'aiguille de ponction (9) de la chemise interne (3) dans la chemise externe (2), où des zones de diamètre agrandi sont prévues préférentiellement à l'extrémité proximale des chemises, et où les mouvements de rota-tion et de coulissement des chemises sont couplés au moyen d'un filet (10) sur ces zones de diamètre agrandi.

7. Canule selon la revendication 6, où ladite canule (1) présente à son extrémité proximale (5) une première section de préhension (18a) raccordée à la chemise interne (3) et une deuxième section de préhension (18b) raccordée à la chemise externe (2), où une rotation relative des sections de préhension entraîne le retrait de toute l'aiguille de ponction (9) de la chemise interne dans la chemise externe (2), et où les fentes longitudinales (6, 7) de la chemise interne et de la chemise externe viennent à se superposer.

8. Canule selon l'une des revendications précédentes, où l'extrémité distale (4) de la chemise externe est sensiblement émoussée.

9. Canule selon l'une des revendications précédentes, réalisée de telle manière qu'une rétro-rotation complète de la chemise interne (3) vers la première position depuis la deuxième position est empêchée, et où la chemise interne (3) s'élargit préférentiellement en deuxième position, ce qui rend impossible une rétro-rotation vers la première position.

10. Canule selon l'une des revendications précédentes, où la surface intérieure de la chemise externe (2) présente deux éléments de stabilisation (14) à son extrémité distale (4), lesquels stabilisent la chemise interne (3) lors de la ponction.

11. Canule selon l'une des revendications précédentes, où une troisième position de rotation est prévue entre la première et la deuxième position de rotation, où l'aiguille de ponction (9) est déjà entièrement rétractable dans la chemise externe (3), les fentes longitudinales (6, 7) de la chemise interne et de la chemise externe ne venant toutefois pas encore à se superposer, la canule étant préférentiellement réalisée de manière à empêcher une rétro-rotation complète de la chemise interne (3) vers la première position depuis la troisième position, et/ou où une rotation de la chemise interne (3) vers la troisième position entraîne préférentiellement la rétractabilité complète de l'aiguille de ponction (9) de la chemise interne (3) dans la chemise externe (2).

12. Canule selon la revendication 11, où ladite canule (1) présente à son extrémité proximale (5) une première section de préhension (18a) raccordée à la chemise interne (3) et une deuxième section de préhension (18b) raccordée à la chemise externe (2), où une rotation relative des sections de préhension entraîne le retrait de toute l'aiguille de ponction (9) de la chemise interne dans la chemise externe (2) en troisième position de rotation, où les fentes longitudinales (6, 7) de la chemise interne et de la chemise externe viennent à se superposer en deuxième position de rotation, et où un filet (10) est préférentiellement prévu sur les sections de préhension (18a, 18b), au moyen duquel les mouvements de rotation et de coulissement des chemises sont couplés.

13. Canule selon la revendication 7 ou la revendication 12, où la chemise externe (2) et la deuxième section de préhension (18b) sont fabriquées d'un seul tenant dans une matière plastique.

14. Canule selon l'une des revendications 5, 7, 12 et 13, où la section de préhension (8a ; 18a) raccordée à la chemise interne (3) comprend une plaque de pression.

15. Kit avec une canule (1) selon l'une des revendications précédentes et un cathéter (11) pouvant être logé dans la chemise interne (3) de la canule (1), où le cathéter (11) peut en deuxième position de canule être retiré de la canule (1) par les fentes longitudinales (6, 7) superposées des chemises (2, 3).
